# EUROPEAN PATENT SPECIFICATION

(11) **EP 4 291 156 B1**
(45) Date of publication and mention of the grant of the patent: **22.01.2025**
(21) Application number: 22704378.3
(22) Date of filing: 08.02.2022
(51) Int. Cl.: A61K 8/37, A61Q 13/00, C07C 69/533, C07C 69/56, C07C 319/18, G01N 30/88

(54) **PROCESS FOR PREPARING A THIOETHER**
VERFAHREN ZUR HERSTELLUNG EINES THIOETHERS
PROCÉDÉ DE PRÉPARATION D'UN THIOÉTHER

(30) Priority: 11.02.2021 US 202163148182 P; 22.02.2021 EP 21158321
(43) Date of publication of application: 20.12.2023
(73) Proprietor: Firmenich SA, 1242 Satigny (CH)
(72) Inventor: ODDON, Gilles, 1242 Satigny (CH); FREROT, Eric, 1242 Satigny (CH); BOSHRA, Ramez, Newark, New Jersey 07114 (US)
(74) Representative: Strych, Sebastian
(86) International application number: PCT/EP2022/053012
(87) International publication number: WO 2022/171627

(56) References cited:
- WO-A1-2017/139920
- US-A1- 2013 336 914
- CHARLES FEHR ET AL: "Aldols by Michael Addition: Application of the retro-Michael Addition to the Slow Release of Enones", HELVETICA CHIMICA ACTA, vol. 88, no. 12, 1 December 2005 (2005-12-01), pages 3128 - 3136, XP055034584, ISSN: 0018-019X, DOI: 10.1002/hlca.200590252
- DAMIEN L. BERTHIER ET AL: "Influence of the Backbone Structure on the Release of Bioactive Volatiles from Maleic Acid-Based Polymer Conjugates", BIOCONJUGATE CHEMISTRY, vol. 21, no. 11, 17 November 2010 (2010-11-17), pages 2000 - 2012, XP055034586, ISSN: 1043-1802, DOI: 10.1021/bc100223s
- UMBERTO MADDALENA ET AL: "Thioether Profragrances: Parameters Influencing the Performance of Precursor-Based Fragrance Delivery in Functional Perfumery", CHEMISTRY & BIODIVERSITY, vol. 11, no. 11, 1 November 2014 (2014-11-01), CH, pages 1700 - 1733, XP055502263, ISSN: 1612-1872, DOI: 10.1002/cbdv.201400023

## Description

### Technical field

The present invention relates to the field of organic synthesis and, more specifically, it concerns a process for preparing a compound of formula (I). Moreover, the present invention comprises also analytical methods of measuring the amount of thiol or thioether compounds having less than 6 carbon atoms in the thiol of formula R-SH and a composition of matter comprising at most 50 µg/kg of thiol or thioether compounds having less than 6 carbon atoms and at most 1 ppm of any compound of formula (I').

### Background of the invention

The perfume industry has a particular interest for compositions or additives which are capable of prolonging or enhancing the perfuming effect for a certain period of time. It is particularly desirable to obtain long-lasting properties in order to enhance the consumer experience. Long-lasting perfumes are desirable for various applications, as for example fine or functional perfumery or cosmetic preparations. The washing and softening of textiles are particular fields in which there is a constant need to enable the effect of active substances, in particular perfumes, or perfuming compositions, to be effective for a certain period of time after washing, softening and drying. Indeed, many active substances which are particularly suitable for this type of application are known to lack tenacity on laundry, or do not remain on the laundry when rinsed, with the result that their perfuming effect is experienced only briefly and not very intensely. Given the importance of this type of application in the perfume industry, research in this field has been sustained, in particular with the aim of finding new, and more effective solutions to the aforementioned problems.

Delivery systems have been developed with this regard, such as precursor compounds, also called profragrances or properfumes, which release active material by a chemical reaction during or after application (using O₂, light, enzymes, water (pH) or temperature as the release trigger). Towards this goal, β-thio carbonyl profragrance derivatives of formula (I) herein below described, reported in WO 03/049666, were developed and in particular 3-(dodecylthio)-1-[(1RS,2SR)-2,6,6-trimethyl-3-cyclohexen-1-yl]-1-butanone also known as Haloscent^{®} D (origin: Firmenich SA).

However, the olfactive quality of compound of formula (I) may be significantly affected by traces of impurities undetected by general analytic methods compromising its use in consumer products. In the meantime, compounds of formula (I) can decompose under general purification methods making further purification steps counterproductive.

So there is a need to develop an approach toward compounds of formula (I) while limiting the generation of off-notes and also to develop analytical method allowing to detect impurities present in the starting material and responsible for off-notes in the final product.

The present invention provides a solution to the above problem by preparing compounds of formula (I) by reacting compounds of formula (III) with a thiol of formula R-SH comprising at most 50 µg/kg of thiol or thioether compounds having less than 6 carbon atoms. In addition an analytical method has been developed in order to detect even tiny amount of thiol or thioether compounds having less than 6 carbon atoms.

### Summary of the Invention

The invention relates to a novel process allowing the preparation of compounds of formula (I), through the 1,4-addition (Michael-type addition) of a thiol of formula R-SH on an enal, enone or alpha,beta-unsaturated carboxylic ester of formula (III), without the presence of off-notes while limiting the purification steps leading to the decomposition of compounds of formula (I), and so, leading to a decrease in yield.

So, the first object of the present invention is a method of measuring the amount of thiol or thioether compounds having less than 6 carbon atoms in the thiol of formula R-SH, wherein R represents a linear or branched alkyl group having from 8 to 15 carbon atoms, optionally comprising a carboxylic acid or a C1-8 carboxylic ester functional group, which is not directly linked to the sulfur atom, by Gas-chromatography-Mass Spectrometry (GC-MS) combined with solid phase microextraction (SPME) wherein the SPME is performed with the sample being cooled down to a temperature comprised between -100°C to -50°C.

A second object of the present invention is a process for the preparation of a compound of formula 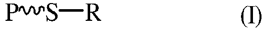
wherein the wavy line indicates the location of the bond between said P and the sulfur atom;
P represents a radical susceptible of generating an odoriferous α,β-unsaturated ketone, aldehyde or carboxylic ester and is represented by the formula 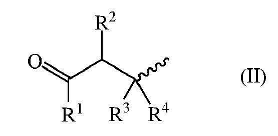
in which the wavy line indicates the location of the bond between said P and S;
R¹ represents a hydrogen atom, a C₁ to C₆ alkoxyl radical or a C₁ to C₁₅ linear, cyclic or branched alkyl, alkenyl or alkadienyl radical, optionally substituted by one to four C₁ to C₄ alkyl groups; and
R², R³ and R⁴ represent independently of each other a hydrogen atom, an aromatic ring, or a C₁ to C₁₅ linear, cyclic or branched alkyl, alkenyl or alkadienyl radical, possibly substituted by C₁ to C₄ alkyl groups; or two, or three, of the groups R¹ to R⁴ are bonded together to form a saturated or unsaturated ring having 5 to 20 carbon atoms and including the carbon atom to which said R¹, R², R³ or R⁴ groups are bonded, this ring being possibly substituted by C₁ to C₈ linear, branched or cyclic alkyl or alkenyl groups;
R represents a linear or branched alkyl group having from 8 to 15 carbon atoms, optionally comprising a carboxylic acid or a C₁₋₈ carboxylic ester functional group which is not directly linked to the sulfur atom;
comprising the step of reacting a compound of formula 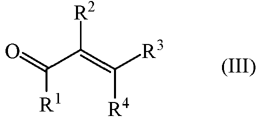
wherein R¹ , R², R³ and R⁴ have the same meaning as defined above with a thiol of formula R-SH wherein R has the same meaning as defined above
characterized in that the thiol comprises at most 50 µg/kg of thiol or thioether compounds having less than 6 carbon atoms.

A third object of the present invention is a composition of matter comprising a compound of formula (I), at most 50 µg/kg of thiol or thioether compounds having less than 6 carbon atoms, and at most 1 ppm of any compound of formula 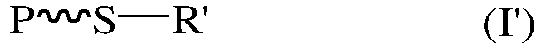 wherein the wavy line indicates the location of the bond between said P and the sulfur atom; P has the same meaning as defined above and R' represents an alkyl group having from 1 to 5 carbon atoms.

### Description of the invention

The invention relates to a novel process for the preparation of compounds of formula (I) devoid of any off-note while maintaining a high yield. The invention process represents an efficient route toward a compound of formula (I) without the presence of impurities badly impacting the organoleptic properties of this compound. Indeed, it has been surprisingly discovered that the presence of thiol or thioether compounds having less than 6 carbon atoms in a thiol of formula R-SH used to prepare compound of formula (I) is responsible for undesired off-notes in the final compound of formula (I) which could not be removed without decreasing the product yield.

So, a first object of the present invention is a process for the preparation of a compound of formula 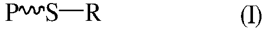 wherein the wavy line indicates the location of the bond between said P and the sulfur atom;
P represents a radical susceptible of generating an odoriferous α,β-unsaturated ketone, aldehyde or carboxylic ester and is represented by the formula 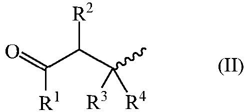
in which the wavy line indicates the location of the bond between said P and S; R¹ represents a hydrogen atom, a C₁ to C₆ alkoxyl radical or a C₁ to C₁₅ linear, cyclic or branched alkyl, alkenyl or alkadienyl radical, optionally substituted by one to four C₁ to C₄ alkyl groups; and
R², R³ and R⁴ represent independently of each other a hydrogen atom, an aromatic ring, or a C₁ to C₁₅ linear, cyclic or branched alkyl, alkenyl or alkadienyl radical, possibly substituted by C₁ to C₄ alkyl groups; or two, or three, of the groups R¹ to R⁴ are bonded together to form a saturated or unsaturated ring having 5 to 20 carbon atoms and including the carbon atom to which said R¹, R², R³ or R⁴ groups are bonded, this ring being possibly substituted by C₁ to C₈ linear, branched or cyclic alkyl or alkenyl groups; R represents a linear or branched alkyl group having from 8 to 15 carbon atoms, optionally comprising a carboxylic acid or a C₁₋₈ carboxylic ester functional group which is not directly linked to the sulfur atom; comprising the step of reacting a compound of formula 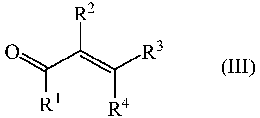 wherein R¹ , R², R³ and R⁴ have the same meaning as defined above with a thiol of formula R-SH wherein R has the same meaning as defined above characterized in that the thiol comprises at most 50 µg/kg of thiol or thioether compounds having less than 6 carbon atoms.

For the sake of clarity, by the expression "thiol or thioether compounds having less than 6 carbon atoms", or the similar, it is meant the normal meaning understood by a person skilled in the art, i.e. an organosulfur compound comprising a -SH functional group and having less than 6 carbon atoms or an organosulfur compound comprising a -S- functional group and having less than 6 carbon atoms.

According to any embodiment of the invention, the invention's process comprises the step of measuring the amount of thiol or thioether compounds having less than 6 carbon atoms in the thiol of formula R-SH by Gas-chromatography-Mass Spectrometry (GC-MS) combined with solid phase microextraction (SPME) wherein the SPME is performed with the neat sample being cooled down to a temperature comprised between -100°C to -50°C. The SPME fiber is preferably a 50/30 µm divinylbenzene/Carboxen on polydimethylsiloxane on a StableFlex fiber (part N°57329-U, Supelco) or 50/30 µm divinylbenzene/Carboxen on polydimethylsiloxane on a 2 cm StableFlex fiber. Alternative fibers that may be used are: 65 µm polydimethylsiloxane/divinylbenzene or 75 µm/85 µm Carboxen/polydimethylsiloxane.

According to any embodiment of the invention, the thiol or thioether compounds having less than 6 carbon atoms may be selected from the group consisting of 1-propanethiol, 2-propanethiol, 2-methyl-1-propanethiol, 2-methyl-2-propanethiol, 1-butanethiol, tetrahydrothiophene and a mixture thereof.

According to any embodiment of the invention, the thiol of formula R-SH comprises
a) at most 5 µg/kg of 1-propanethiol
b) at most 5 µg/kg of 2-propanethiol
c) at most 5 µg/kg of 2-methyl-1-propanethiol
d) at most 20 µg/kg of 2-methyl-2-propanethiol
e) at most 5 µg/kg of 1-butanethiol; and
f) at most 10 µg/kg of tetrahydrothiophene.

In a particular embodiment of the invention, the thiol of formula R-SH comprises
a) at most 2.5 µg/kg of 1-propanethiol
b) at most 3.5 µg/kg of 2-propanethiol
c) at most 2.5 µg/kg of 2-methyl-1-propanethiol
d) at most 18 µg/kg of 2-methyl-2-propanethiol
e) at most 2.5 µg/kg of 1-butanethiol; and
f) at most 5 µg/kg of tetrahydrothiophene.

In a particular embodiment of the invention, the thiol of formula R-SH comprises
a) at most 1 µg/kg of 1-propanethiol
b) at most 3 µg/kg of 2-propanethiol
c) at most 1 µg/kg of 2-methyl-1-propanethiol
d) at most 18 µg/kg of 2-methyl-2-propanethiol
e) at most 1 µg/kg of 1-butanethiol; and
f) at most 3 µg/kg of tetrahydrothiophene.

In a particular embodiment, the thiol of formula R-SH is devoid of traces of 1-propanethiol, 2-propanethiol, 2-methyl-1-propanethiol, 2-methyl-2-propanethiol, 1-butanethiol or tetrahydrothiophene.

According to any embodiment of the invention, the thiol of formula R-SH may be purified by distillation before reacting with the compound of formula (III). In particular, the distillation may be a batch or continuous distillation, in particular, a batch distillation. The distillation may be performed by removing the low boiling point fractions (topping), by selecting the desired fractions (fractional distillation) or by flash distillation using falling (wiped) film evaporators.

According to any embodiment of the invention, R may be a linear alkyl group having from 8 to 15 carbon atoms. In particular R may be a linear alkyl group having from 10 to 14 carbon atoms, and in particular a n-dodecyl group; i.e. R-SH may be 1-dodecanethiol.

As "odoriferous α,β-unsaturated ketone, aldehyde or carboxylic ester", the expression used in the definition of P, it is understood an α,β-unsaturated ketone, aldehyde or carboxylic ester, which is recognized by a skilled person as being used in perfumery as perfuming ingredient. In general, said odoriferous α,β-unsaturated ketone, aldehyde or carboxylic ester is a compound having from 8 to 20 carbon atoms, or even more preferably between 10 and 15 carbon atoms.

Similarly, it is not possible to provide an exhaustive list of the currently known odoriferous compounds of formula (III), which can be used in the synthesis of the compound of formula (I). However, the following can be named as preferred examples: alpha-damascone, beta-damascone, gamma-damascone, delta-damascone, alpha-ionone, beta-ionone, gamma-ionone, delta-ionone, beta-damascenone, 1-[6-ethyl-2,6-dimethyl-3-cyclohexen-1-yl]-2-buten-1-one, 3-methyl-5-propyl-2-cyclohexen-1 -one, 2-methyl-5-(1-propen-2-yl)-2-cyclohexen-1-one, 2,5-dimethyl-5-phenyl-1-hexen-3-one, 1-(5,5-dimethyl-1-cyclohexen-1-yl)-4-penten-1-one, 3,7-dimethylocta-2,6-dienal, 8-methyl-alpha-ionone or 10-methyl-alpha-ionone, 2-octenal, 1-(2,2,3,6-tetramethylcyclohexyl)but-2-en-1-one, 4-(2,2,3,6-tetramethylcyclohexyl)but-3-en-2-one, 2-cyclopentadecen-1-one, 4,4a-dimethyl-6-(1-propen-2-yl)-4,4a,5,6,7,8-hexahydro-2(3H)-naphthalenone, (E)-3-phenylprop-2-enal, 2,6,6-trimethylspiro[bicyclo[3.1.1]heptane-3,1'-cyclohexan]-2'-en-4'-one, ethyl 2,4-decadienoate, ethyl 2-octenoate, methyl 2-nonenoate, ethyl 2,4-undecadienoate, 4-methylpent-3-en-2-one, oct-2-en-4-one, and methyl 5,9-dimethyl-2,4,8-decatrienoate.

According to any embodiment of the invention, the compound of formula (I) may be in a form of any one of its stereoisomers or a mixture thereof and the compound of formula (III) may be in a form of any one of its stereoisomers or a mixture thereof. For the sake of clarity, by the expression "any one of its stereoisomers or a mixture thereof, or the similar, it is meant the normal meaning understood by a person skilled in the art, i.e. that the compound of formula (I) and (III) can be a pure enantiomer or diastereomer. In other words, the compound of formula (I) and (III) may possess several stereocenters and each of said stereocenter can have two different configurations (e.g. R or S). The compound of formula (I) and (III) may even be in the form of a pure enantiomer or in the form of a mixture of enantiomers or diastereoisomers. The compound of formula (I) and (III) can be in a racemic form or scalemic form. Therefore, the compound of formula (I) and (III) can be one stereoisomers or in the form of a composition of matter comprising, or consisting of, various stereoisomers.

According to any one of the above embodiments of the invention, the compound of formula (III) can be in the form of its E or Z isomer or of a mixture thereof, e.g. the invention comprises compositions of matter consisting of one or more compounds of formula (III), having the same chemical structure but differing by the configuration of the double bond.

According to any embodiment of the invention, P is a radical selected from the group consisting of formulae (P-1) to (P-14), in the form of any one of their isomers: 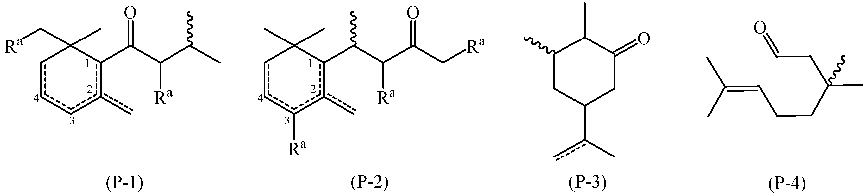 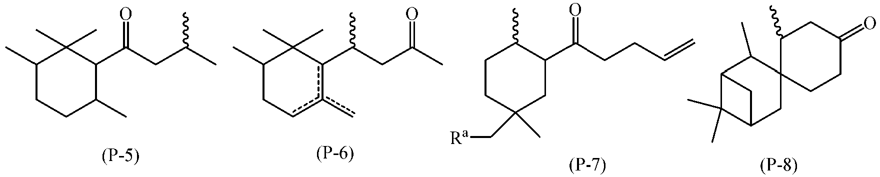 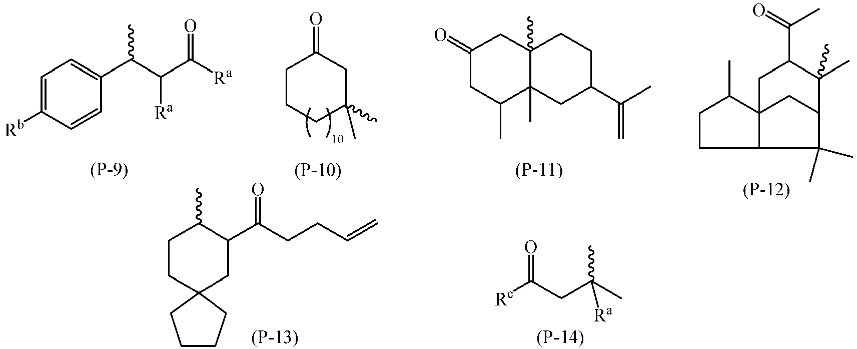 in which formulae the wavy lines have the meaning indicated above and the dotted lines represent a single or double bond, R^{a} being a hydrogen atom or a methyl group and R^{b} representing a hydrogen atom, a hydroxyl or methoxy group or a C₁-C₄ linear or branched alkyl group and R^{c} representing a hydrogen atom or a C₁-C₄ linear or branched alkyl group. Accordingly, the compound of formula (I) wherein P is selected from the group consisting of formulae (P-1) to (P-14) is prepared by reacting compound of formula 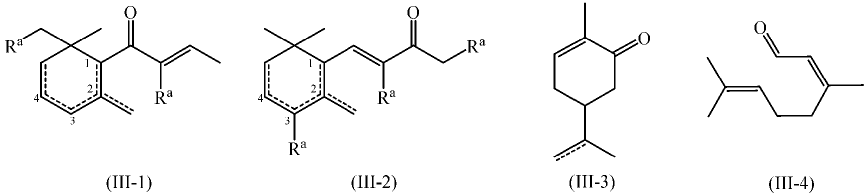 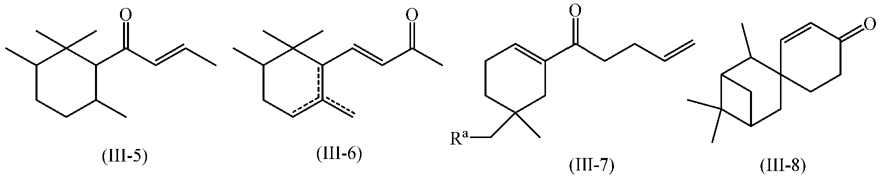 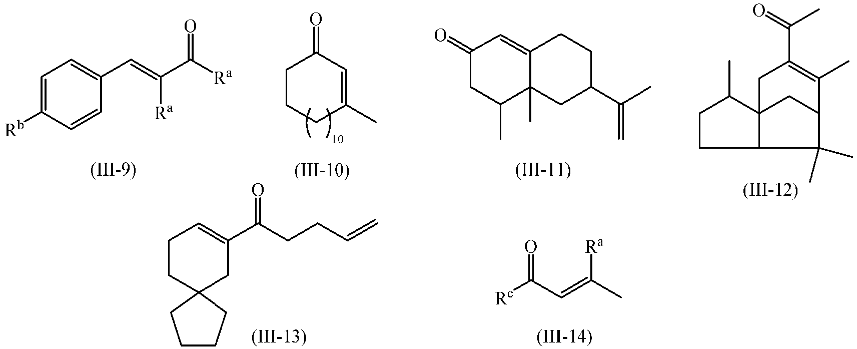 wherein the dotted lines, R^{a}, R^{b} and R^{c} have the same meaning as defined above.

For the sake of clarity, by the expression "wherein one dotted line represents a single or double bond", or the similar, it is meant the normal meaning understood by a person skilled in the art, i.e. that the whole bonding (solid and dotted line) between the carbon atoms connected by said dotted line is a carbon-carbon single bond or a carbon-carbon double bond.

In a particular embodiment, P represents a radical selected from the group consisting of formulae 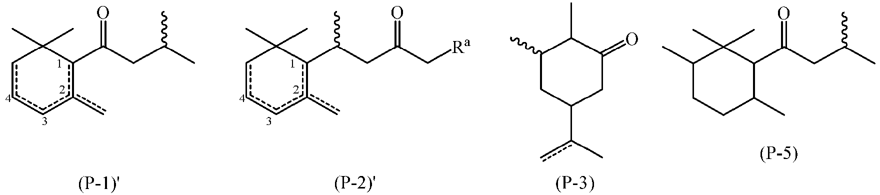 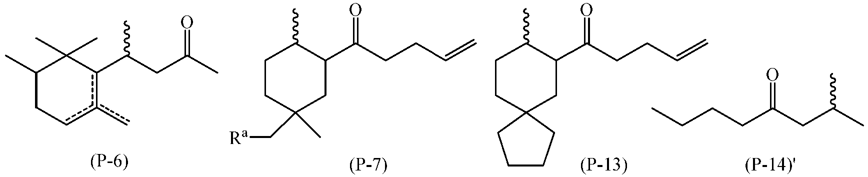 wherein the wavy lines have the meaning indicated above and the dotted lines represent a single or double bond, and R^{a} being a hydrogen atom or a methyl group. Accordingly, the compound of formula (I) wherein P is selected from the group consisting of formulae (P-1)', (P-2)', (P-3), (P-5), (P-6), (P-7), (P-13) and (P-14)' is prepared by reacting compound of formula 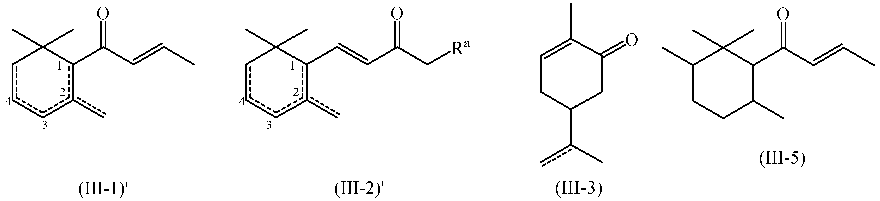 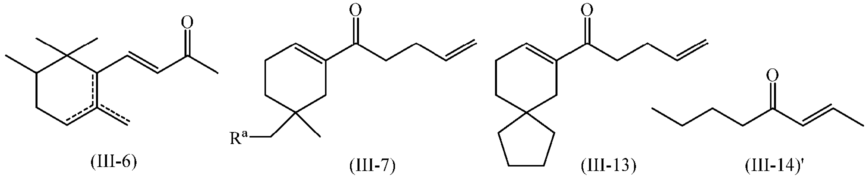 wherein the dotted lines and R^{a} have the same meaning .as defined above.

In a particular embodiment, P represents a radical selected from the group consisting of formulae (P-1), (P-2), (P-1)', (P-2)', (P-3), (P-7), (P-13), (P-14) or (P-14)' as defined above. In particular, P may be (P-1), (P-2), (P-3), (P-7), (P-13) or (P-14). Preferably, P represents a radical selected from the group consisting of formulae (P-1), (P-1)', (P-2), (P-2)', (P-3) or (P-14)' as defined above.

In a particular embodiment, the compound of formula (I) is a compound selected from the group consisting of formulae a) to d) 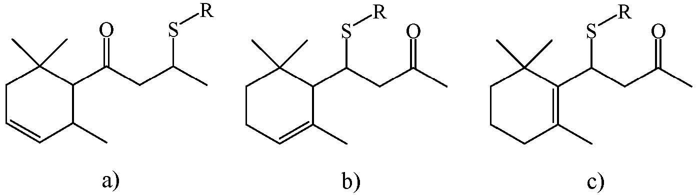 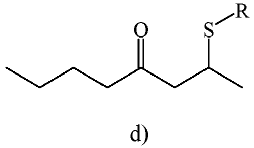 wherein R has the same menaing as defined above.

The compound of formula a) is obtained from the reaction of a compound of formula (III), wherein the compound of formula (III) is delta-damascone. Said compound of formula a) may preferably be (±)-trans-3-(dodecylthio)-1-(2,6,6-trimethyl-3-cyclohexen-1-yl)-1-butanone. Delta-damascone is also known as 1-[(1RS,2SR)-2,6,6-trimethyl-3-cyclohexen-1-yl]-2-buten-1-one.

The compound of formula b) or c) is obtained from the reaction of a compound of formula (III), wherein the compound of formula (III) is ionone. Said compound of formula b) or c) may be present as an isomeric mixture of formula b) and formula c). The isomeric mixture may have a weight ratio of formula b) and formula c) from 40:60 to 60:40. In particular, the isomeric mixture may have a weight ratio of formula b) and formula c) of about 55:45. In particular, said compound of formula b) or c) releases two isomers of ionone as fragrance compound.

In particular, the compound of formula b) is obtained from the reaction of a compound of formula (III), wherein the compound of formula (III) is alpha-ionone. Said compound of formula b) may preferably be (±)-4-(dodecylthio)-4-(2,6,6-trimethyl-2-cyclohexen-1-yl)-2-butanone. Alpha-ionone is also known as (±)-(3E)-4-(2,6,6-trimethyl-2-cyclohexen-1-yl)-3-buten-2-one.

In particular, the compound of formula c) is obtained from the reaction of a compound of formula (III), wherein the compound of formula (III) is beta-ionone. Said compound of formula c) may preferably be (±)-4-(dodecylthio)-4-(2,6,6-trimethyl-1-cyclohexen-1-yl)-2-butanone. Beta-ionone is also known as (3E)-4-(2,6,6-trimethyl-1-cyclohexen-1-yl)-3-buten-2-one.

The compound of formula d) is obtained from the reaction of a compound of formula (III), wherein the compound of formula (III) is oct-2-en-4-one. Said compound of formula d) may preferably be (±)-2-(dodecylthio)octan-4-one. Oct-2-en-4-one may be used as its (E)- or (Z)- isomers, or as mixtures thereof, with the (E)-isomer being preferred.

According to any embodiment of the invention, and as non-limiting examples of compounds of formula (I) one may cite the following: 3-(dodecylthio)-1-(2,6,6-trimethylcyclohex-3-en-1-yl)butan-1-one, 2-(dodecylthio)-4-octanone, 3-(dodecylthio)-1-(2,6,6-trimethylcyclohex-2-en-1-yl)butan-1-one, 4-(dodecylthio)-4-(2,6,6-trimethylcyclohex-2-en-1-yl)butan-2-one and 4-(dodecylthio)-4-(2,6,6-trimethylcyclohex-1-en-1-yl)butan-2-one and 4-(dodecylthio)-4-methylpentan-2-one.

According to any embodiment of the invention, the reaction between the compound of formula (III) and the thiol of formula R-SH may be conducted in the presence of any reagent commonly used for 1,4-additions such as a base, Lewis acid, transitional metal or organocatalyst. Particularly, said reaction may be performed in presence of a base. Particularly, the base is a tertiary amine. Non-limiting examples of suitable bases may include triethylamine, trimethylamine, urotropine, N,N-diisopropylethylamine, 1,8-diazobicyclo(5.4.0)undec-7-ene, or a mixture thereof.

The base can be added into the reaction medium of the invention's process in a large range of concentrations to form a compound of formula (I). As non-limiting examples, one can cite, as base concentration values those ranging from 0.001 to 2 equivalents, relative to the total amount of compound of formula (III). Particularly, the base concentration may be comprised between 0.005 to 1.5 equivalents relative to the total amount of compound of formula (III). It goes without saying that the process works also with more base. However the optimum concentration of base will depend, as the person skilled in the art knows, on the nature of the latter, on the nature of compound of formula (III) and of thiol of formula R-SH, on the temperature and on the desired time of reaction.

The thiol of formula R-SH can be added into the reaction medium of the invention's process in a large range of concentrations to form compound of formula (I). As non-limiting examples, one can cite, as thiol concentration values those ranging from 0.5 to 5 equivalents, relative to the total amount of compound of formula (III). Particularly, the thiol concentration may be comprised between 0.75 to 3 equivalents relative to the total amount of compound of formula (III). It goes without saying that the process works also with more thiol of formula R-SH. However the optimum concentration of thiol of formula R-SH will depend, as the person skilled in the art knows, on the nature of the latter, on the nature of compound of formula (III) and of base, on the temperature and on the desired time of reaction.

According to any one of the invention's embodiments, the invention's process to form a compound of formula (I) is carried out at a temperature comprised between -10°C and 150°C. In particular, the temperature is in the range between 30°C and 70°C. Of course, a person skilled in the art is also able to select the preferred temperature as a function of the melting and boiling point of the starting and final products as well as the desired time of reaction or conversion.

The invention's process to form a compound of formula (I) can be carried out in the presence or absence of a solvent. When a solvent is required or used for practical reasons, then any solvent current in such reaction type can be used for the purposes of the invention. Non-limiting examples include C₆₋₁₂ aromatic solvents such as xylene, toluene, 1,3-diisopropylbenzene, cumene or pseudocumene, or mixtures thereof, hydrocarbon solvents such as cyclohexane, heptane or mixtures thereof, nitrile solvents such as acetonitrile, esteral solvents such as ethyl acetate or ethereal solvents such as tetrahydrofuran, diethyether, methyl tetrahydrofuran or mixtures thereof. The choice of the solvent is a function of the nature of the substrates and/or base and the person skilled in the art is well able to select the solvent most suitable in each case to optimize the reaction. Particularly, the invention's process to form a compound of formula (I) is carried out in absence of solvent.

The invention's process to form a compound of formula (I) is carried out under batch, semi-batch or continuous conditions.

The invention's process to form a compound of formula (I) may further comprise the step of purifying the compound of formula (I). The person skilled in the art is well aware of the most adapted purification method. Non-limiting examples of suitable purification methods may include washing-drying, vacuum distillation in particular vacuum distillation using wiped-film distillation or chromatography.

The presence of a tiny amount of sulfur-containing hydrocarbons impurities present in the compound of formula (I), which are responsible for undesirable off-notes in the final product, has to be minimized. It has been surprisingly discovered that there is a link between the presence of thiol or thioether compounds having less than 6 carbon atoms in the thiol of formula R-SH and the presence of off-notes in the final product, which could surprisingly not be removed by purification at the end of the process. The detection of thiol or thioether compounds having less than 6 carbon atoms in the thiol of formula R-SH cannot be performed using a simple GC method since it is not be able to detect these very powerful odorants with the relevant sensitivity. A novel analytical method has been developed for this specific purpose.

So another object of the invention is a method of measuring the amount of thiol or thioether compounds having less than 6 carbon atoms in the thiol of formula R-SH, wherein R has the same meaning as defined above, by Gas-chromatography-Mass Spectrometry (GC-MS) combined with solid phase microextraction (SPME) wherein the SPME is performed with the neat sample cooled down to a temperature comprised between -100°C to -50°C. The SPME fiber is a 50/30µm DVB/CAR/PDMS Stableflex, (part N°57329-U, Supelco).

According to any embodiment of the invention, the thiol or thioether compounds having less than 6 carbon atoms detected by this method are selected from the group consisting of 1-propanethiol, 2-propanethiol, 2-methyl-1-propanethiol, 2-methyl-2-propanethiol, 1-butanethiol and tetrahydrothiophene.

According to any embodiment of the invention, the MS of the GC/MS is a triple quadrupole, a quadrupole Time-of-Flight (Q-TOF) or a quadrupole orbitrap. The gas used is helium.

According to any embodiment of the invention, the mode of acquiring GC-MS data is Selected Ion Monitoring (SIM) or Selected Reaction Monitoring (SRM).

According to a particular embodiment, the GC chromatography uses a non-polar GC column (DB-1, SPB-1) or possibly a GC column of moderate polarity (DB-5). The GC gradient oven has to start at low temperature so as to separate the volatile sulfur compounds adequately.

A further object of the invention is a composition of matter comprising a compound of formula (I), at most 50 µg/kg of thiol or thioether compounds having less than 6 carbon atoms and at most 1 ppm of any compound of formula 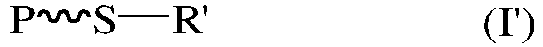 wherein the wavy line indicates the location of the bond between said P and the sulfur atom; P has the same meaning as defined above R' represents an alkyl group having from 1 to 5 carbon atoms.

The analytical method used to measure the amount of any product of formula (I') in the compound of formula (I) is HPLC/MS or UPLC/MS. The product of formula (I) is typically injected at a concentration of up to 10% onto a C₁₈ column eluted with water/acetonitrile. The flow to the MS is typically diverted when the major product (I) elutes which is always at retention times higher than those of smaller products of formula (I'). The MS may be selected from the group consisting of triple quadrupole, Q-Tof and Q-Orbitrap. When the signal corresponding to the molecular ion of (I') is not seen, the corresponding product is generally below 10 µg/kg.

### Examples

The invention will now be described in further detail by way of the following examples, wherein the abbreviations have the usual meaning in the art, the temperatures are indicated in degrees centigrade (°C). The solvents were dried by conventional procedures and distilled under an argon atmosphere. NMR spectra were recorded at 20 °C on Bruker AV 300, AV 400, or AV 500 MHz spectrometers. Chemical shifts are reported in ppm relative to solvent signals (chloroform, δ_{H} = 7.26 ppm, δ_{C} = 77.0 ppm). The signal assignment was ensured by recording ¹H,¹H- COSY, -NOESY, ¹³C,¹H-HSQC and -HMBC experiments.

### Example 1

### Measurement of the amount of thiol or thioether having less than 6 carbon atoms in dodecanethiol

3 batches of dodecanethiol have been analyzed by solid-phase microextraction (SPME) and GC/MS as described below. Batch 3 was obtained by distillation of batch 2 as reported below.

### a) Typical dodecanethiol distillation procedure

600 g of dodecanethiol were charged into a round bottom flask equipped with a thermowell, magnetic stir bar and connected to a fractionation column. The fractionation column was filled with a structured packing with an efficiency of approx. 15 theoretical plates. The distillation commenced under reduced pressure (2-3 mmHg) and the pot was heated until a maximum temperature of 161 °C. The following fractions were obtained.

| **T(pot) (°C)** | **T(vap) (°C)** | **Pressure (mmHg)** | **wt (g)** | |
|---|---|---|---|---|
| 134 | 112 | 2.3 | 134.8 | front fraction |
| 135 | 114 | 2.2 | 325 | heart fraction |
| 161 | 87 | 2.2 | 95.6 | heart fraction |
| 161 | - | 2.2 | 38 | heavy fraction |

### b) SPME method

SPME was performed using a grey fiber (50/30µm DVB/CAR/PDMS Stableflex, part N°57329-U, Supelco). Manual SPME extractions at -80°C (15 min) were performed by manual swirling a 20 mL headspace vial containing dodecanethiol (1 g) in a dry ice/ethanol bath until dodecanethiol solidified on the glass wall of the vial. Then the fiber was exposed to the headspace of the sample for 15 min while the vial was still partially immerged in the dry ice/ethanol bath. This SPME method assured the highest sensitivity.

Alternatively, SPME can be performed at RT using an automatic sampler (combi-PAL, CTC) but with a lower sensitivity.

### c) GC/MS method

### A 7890/7000B triple quadrupole GC/MS system (Agilent) was used. The column was a

SPB-1, 30 m, 0.25 mm ID, 0.25 µm film thickness (Supelco) eluted with He as a carrier gas at 0.7 mL/min, Oven: 40°C, 5 min then 5°C/min to 60 °C and finally 20 °C/min to 250°C, 250°C for 1.5 min. The parent/product ions and collision energy (expressed in eV) for the different products were as followed:
1-butanethiol: 90/56 (3 eV) and 90/41 (6 eV); 1-propanethiol: 76/60 (3 eV) and 76/47 (6 eV); 2-methyl-1-propanethiol: 90/56 (4 eV) and 90/41 (6 eV); 2-propanethiol: 76/60 (2 eV) and 76/43 (6 eV); 2-methyl-2-propanethiol: 90/57 (2 eV) and 90/41 (2 eV); tetrahydrothiophene: 88/60 (14 eV) and 88/54 (9 eV)

The SPME protruding fiber was exposed for 5 min before GC/MS injection using the autosampler PAL automatized procedure. Splitless injection was used.

### d) Results

**Table 1 : Measurement of thiol or thioether compounds having less than 6 carbon atoms in dodecanthiol**

| Thiols content expressed in µg/kg | Batch 1 | Batch 2 | Batch 3¹⁾ front fraction | Batch 3¹⁾ heart fraction | Batch 3¹⁾ heavy fraction |
|---|---|---|---|---|---|
| 1-butanethiol | 0 | 19 | 129 | 34 | 0 |
| 1-propanethiol | 0 | 153 | 45 | 2 | 0 |
| 2-methyl-1 -propanethiol | 0 | 4 | 1 | 1 | 1 |
| 2-propanethiol | 3 | 142 | 26 | 3 | 2 |
| 2-methyl-2-propanethiol | 18 | 523 | 142 | 13 | 3 |
| Tetrahydrothiophene | 3 | 4834 | 4508 | 183 | 2 |

### 1) Batch 3 obtained by distillation of batch 2. front, heart and heavy fractions

Batch 1 and the heavy fractions of Batch 3 represent dodecanethiol batches to be used in the invention process. Batch 2 and the front and heart fractions of Batch 3 represent dodecanethiol batches to be used to prepare comparative examples.

### Example 2

### Preparation of a compound of formula (I) and analysis

### a) Preparation of compound of formula (I)

A solution of δ-damascone (10.0 g; 52.1 mmol) and 1-dodecanethiol using 5 different batches as reported below (8.42 g, 41.7 mmol) in THF (150 ml) was treated with DBU (7.92 g; 52.1 mmol) and stirred at 45°C for 90 min. The reaction solution was treated with 5% aqueous HCl, extracted twice with ether, washed with H₂O, saturated aqueous NaHCO₃, then brine, dried over Na₂SO₄, and concentrated at 70°C/0.01 mbar. Yield of crude product: 16.2 g (99%).

¹H-NMR: 0.84-0.92 (m, 6 H); 0.93-1.02 (4 s, 6 H); 1.26 (m, 16 H); 1.29 (m, 3 H); 1.36 (m, 2 H); 1.58 (m, 2 H); 1.69 (m, 1H); 1.96 (2 b, 1 H); 2.22 (m, 1 H); 2.50 (m, 3.5 H); 2.70 (m, 1 H); 2.90 (m, 0.5 H); 3.30 (m, 1 H); 5.43 (m, 1 H); 5.53 (m, 1 H).

¹³C-NMR: 212.4/5 (s); 131.8/9 (d); 124.1/2 (d); 62.9/63.0 (d); 55.2/3 (t); 41.7 (t); 34.1 (d); 33.0/2 (s); 31.9 (t); 31.6/8 (d); 30.9 (t); 29.8 (q); 29.0-29.8 (several t); 22.7 (t); 21.6/8 (q); 20.7 (q); 19.9 (q); 14.1 (q).

### b) Measurement of the amount of thiol or thioether having less than 6 carbon atoms in the products of formula (I)

The measurement of the amount of thiols having less than 6 carbon atoms in the products of formula (I) has been performed using the SPME GC/MS method described in example 1. The SPME sampling of (I) was performed at room temperature. The results are shown in Table 2.

All batches of (I) obtained with different dodecanethiol batches have also been analyzed by UPLC/MS to assess their content in compounds of formula (I'). The system was composed of an Acquity I-Class UPLC system (Waters) coupled to a QExactive Plus mass spectrometer (Thermo). The column was an Acquity BEH C18 (1.7 µm, 2.1 x 100 mm) eluted with 0.4 ml/min of H₂O/ACN 0.1% formic acid. The gradient was as follows: 0% B, 0.5 min ; 0-100% B in 10 min ; 100% B, 1 min ; equilibration for 1 min. The MS was operated in the negative ESI mode, voltage 3500V, probe heater (source) 300°C, capillary 300°C, sheath gas 48, aux gas 11. Full scan ddMS2 mode [150-2000 Da] was used for qualitative and quantitative analyses with stepped NCEs of 10, 30 and 45V. PRM by using an inclusion list and isolation width of 1.0 Da (no offset). The results are reported in Table 2.

**Table 2: Measurement of thiol or thioether compounds having less than 6 carbon atoms and compounds of formula (I') in 3-(dodecylthio)-1-[2,6,6-trimethyl-3-cyclohexen-1-yl]-1-butanone**

| Thiols content expressed in µg/kg (SPME GC/MS method) | Batch 1 | Batch 2 | Batch 3 front fraction |
|---|---|---|---|
| 1-butanethiol | <1 | <1 | <1 |
| 1-propanethiol | <1 | <1 | <1 |
| 2-methyl- 1-propanethiol | <1 | <1 | <1 |
| 2-propanethiol | <1 | <1 | <1 |
| 2-methyl-2-propanethiol | <1 | <1 | 1-4 |
| Tetrahydrothiophene | <1 | 390 | 1575 |
| Products of formula (I') (UPLC/MS method) | No (I') detected | Propyl and butyl (I') detected | Not analyzed |

The presence of 1-propanethiol, 2-propanethiol, 2-methyl-1-propanethiol, 2-methyl-2-propanethiol, 1-butanethiol was not detected in the final product even though the compound of formula (I) prepared with those batches possess strong off-notes as shown below. Those results confirm that the analysis of small thiols has to be performed beforehand on the starting material dodecanethiol. Indeed, even undetected traces of those compound in the final product is detrimental.

### Example 3

### Organoleptic evaluation of compounds of formula (I)

The batches obtained in Example 2 have been evaluated by three trained panelists by smelling above the freshly open bottle. The panelists were asked to rate the sulfur odor intensity on a scale from 1 to 10, 1 corresponding to odorless and 10 corresponding to a very strong odor.

The results are reported in Table 3.

**Table 3: Organoleptic evaluation of three batches of 3-(dodecylthio)-1-[2,6,6-trimethyl-3-cyclohexen-1-yl]-1-butanone prepared in example 2**

| From dodecanthiol | Batch 1 | Batch 2 | Batch 3 front fraction | Batch 3 Heart fraction | Batch 3 Heavy fraction |
|---|---|---|---|---|---|
| Sulfur odor Intensity | 3 | 7 | 9 | 5 | 3 |

Only compound of formula (I) prepared with batches 1 and heavy fraction of batch 3 of dodecanethiol imparted a very low undesired sulfur odor.

## Claims

1. A method of measuring the amount of thiol or thioether compounds having less than 6 carbon atoms in the thiol of formula R-SH, wherein R represents a linear or branched alkyl group having from 8 to 15 carbon atoms, optionally comprising a carboxylic acid or a C₁₋₈ carboxylic ester functional group, which is not directly linked to the sulfur atom, by Gas-chromatography-Mass Spectrometry (GC-MS) combined with solid phase microextraction (SPME) wherein the SPME is performed with the sample cooled down to a temperature comprised between -100°C to -50°C.

2. The method according to claim 1, wherein the thiol or thioether compounds having less than 6 carbon atom are selected from the group consisting of 1-propanethiol, 2-propanethiol, 2-methyl-1-propanethiol, 2-methyl-2-propanethiol, 1-butanethiol and tetrahydrothiophene.

3. The method according to any one of claims 1 to 2, wherein the MS of the GC/MS is a triple quadrupole, a quadrupole Time-of-Flight (Q-TOF) or a quadrupole orbitrap.

4. The method according to any one of claims 1 to 3, wherein the mode of acquiring GC-MS data is Selected Ion Monitoring (SIM) or Selected Reaction Monitoring (SRM).

5. A process for the preparation of a compound of formula 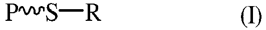
wherein the wavy line indicates the location of the bond between said P and the sulfur atom;
P represents a radical susceptible of generating an odoriferous α,β-unsaturated ketone, aldehyde or carboxylic ester and is represented by the formula 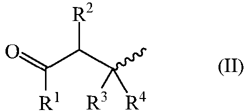 in which the wavy line indicates the location of the bond between said P and S; R¹ represents a hydrogen atom, a C₁ to C₆ alkoxyl radical or a C₁ to C₁₅ linear, cyclic or branched alkyl, alkenyl or alkadienyl radical, optionally substituted by one to four C₁ to C₄ alkyl groups; and
R², R³ and R⁴ represent independently of each other a hydrogen atom, an aromatic ring, or a C₁ to C₁₅ linear, cyclic or branched alkyl, alkenyl or alkadienyl radical, possibly substituted by C₁ to C₄ alkyl groups; or two, or three, of the groups R¹ to R⁴ are bonded together to form a saturated or unsaturated ring having 5 to 20 carbon atoms and including the carbon atom to which said R¹, R², R³ or R⁴ groups are bonded, this ring being possibly substituted by C₁ to C₈ linear, branched or cyclic alkyl or alkenyl groups; R represents a linear or branched alkyl group having from 8 to 15 carbon atoms, optionally comprising a carboxylic acid or a C₁₋₈ carboxylic ester functional group which is not directly linked to the sulfur atom;
comprising the step of reacting a compound of formula 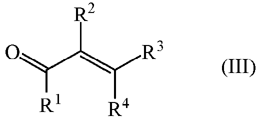 wherein R¹, R², R³ and R⁴ have the same meaning as defined above with a thiol of formula R-SH wherein R has the same meaning as defined above **characterized in that** the thiol comprises at most 50 µg/kg of thiol or thioether compounds having less than 6 carbon atoms.

6. The process according to claim 5, wherein the thiol of formula R-SH comprises
a) at most 5 µg/kg of 1-propanethiol
b) at most 5 µg/kg of 2-propanethiol
c) at most 5 µg/kg of 2-methyl-1-propanethiol
d) at most 20 µg/kg of 2-methyl-2-propanethiol
e) at most 5 µg/kg of 1-butanethiol; and
f) at most 10 µg/kg of tetrahydrothiophene.

7. The process according to any one of claims 5 to 6, wherein the thiol of formula R-SH is devoid of traces of 1-propanethiol, 2-propanethiol, 2-methyl-1-propanethiol, 2-methyl-2-propanethiol, 1-butanethiol or tetrahydrothiophene.

8. The process according to any one of claims 5 to 7, wherein the thiol of formula R-SH is purified by distillation before reacting with the compound of formula (III).

9. The process according to any one of claims 5 to 8, wherein R can be a linear alkyl group having from 10 to 14 carbon atoms.

10. The process according to any one of claims 5 to 9, wherein the thiol of formula R-SH is 1-dodecanethiol.

11. The process according to any one of claims 5 to 10, wherein P is a radical selected from the group consisting of formulae (P-1) to (P-14), in the form of any one of their isomers: 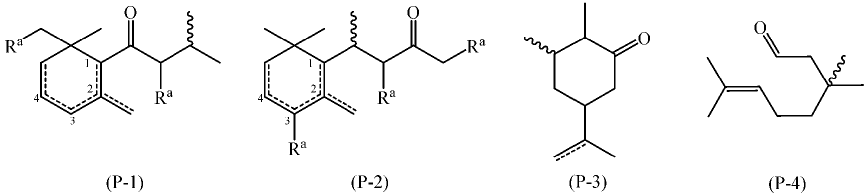 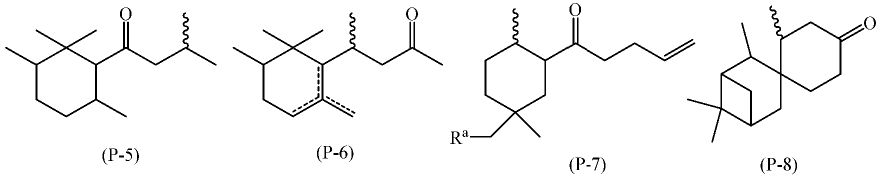 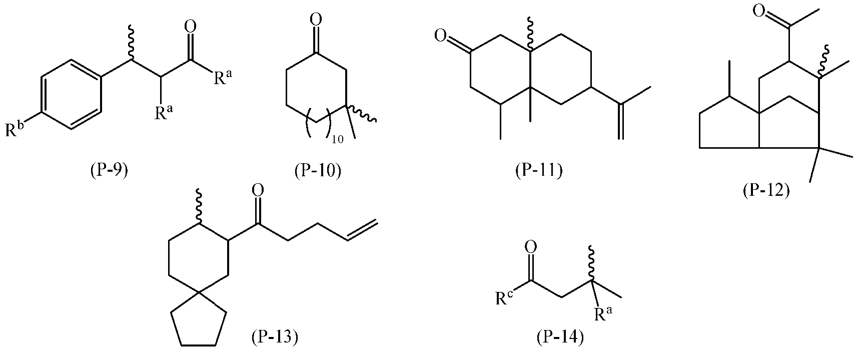 in which formulae the wavy lines have the meaning indicated above and the dotted lines represent a single or double bond, R^{a} being a hydrogen atom or a methyl group and R^{b} representing a hydrogen atom, a hydroxyl or methoxy group or a C₁-C₄ linear or branched alkyl group and R^{c} representing a hydrogen atom or a C₁-C₄ linear or branched alkyl group.

12. The process according to any one of claims 5 to 11, wherein P is (P-1), (P-2), (P-3), (P-7), (P-13) or (P-14).

13. The process according to any one of claims 5 to 12, wherein compound of formula (I) is selected from the group of 3-(dodecylthio)-1-(2,6,6-trimethylcyclohex-3-en-1-yl)butan-1-one, 2-(dodecylthio)-4-octanone, 3-(dodecylthio)-1 -(2,6,6-trimethylcyclohex-2-en-1-yl)butan-1-one, 4-(dodecylthio)-4-(2,6,6-trimethylcyclohex-2-en-1-yl)butan-2-one and 4-(dodecylthio)-4-(2,6,6-trimethylcyclohex-1-en-1-yl)butan-2-one and 4-(dodecylthio)-4-methylpentan-2-one.

14. The process according to any one of claims 5 to 13, wherein the reaction between compound of formula (III) and the thiol of formula R-SH is conducted in the presence of a base.

15. A composition of matter comprising compound of formula (I), at most 50 µg/kg of thiol or thioether compounds having less than 6 carbon atoms, and at most 1 ppm of any compound of formula 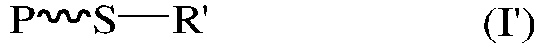 wherein the wavy line indicates the location of the bond between said P and the sulfur atom; P has the same meaning as defined in claims 5 to 14 and R' represent alkyl group having from 1 to 5 carbon atoms.

## Patentansprüche

1. Methode zur Messung der Menge an Thiol- oder Thioetherverbindungen mit weniger als 6 Kohlenstoffatomen im Thiol der Formel R-SH, wobei R eine geradkettige oder verzweigte Alkylgruppe mit 8 bis 15 Kohlenstoffatomen darstellt, die gegebenenfalls eine Carbonsäure- oder eine C₁₋₈-Carbonsäureester-Funktionsgruppe umfasst, die nicht direkt mit dem Schwefelatom verknüpft ist, durch Gaschromatographie-Massenspektrometrie (GC-MS) in Kombination mit Festphasen-Mikroextraktion (Solid Phase Microextraction, SPME), wobei die SPME unter Kühlung der Probe auf eine Temperatur zwischen -100°C bis -50°C durchgeführt wird.

2. Methode nach Anspruch 1, wobei die Thiol- oder Thioetherverbindungen mit weniger als 6 Kohlenstoffatomen aus der Gruppe bestehend aus 1-Propanthiol, 2-Propanthiol, 2-Methyl-1-propanthiol, 2-Methyl-2-propanthiol, 1-Butanthiol und Tetrahydrothiophen ausgewählt sind.

3. Methode nach einem der Ansprüche 1 bis 2, wobei es sich bei der MS der GC/MS um eine Triple-Quadrupole-, eine Quadrupole-Time-of-Flight- (Q-TOF-) oder eine Quadrupole-Orbitrap-MS handelt.

4. Methode nach einem der Ansprüche 1 bis 3, wobei die Erfassung von GC-MS-Daten im SIM(Selected Ion Monitoring)- oder SRM(Selected Reaction Monitoring)-Modus erfolgt.

5. Verfahren zur Herstellung einer Verbindung der Formel 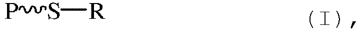
wobei die Wellenlinie die Lage der Bindung zwischen dem P und dem Schwefelatom kennzeichnet;
P einen Rest darstellt, der zur Erzeugung eines α,β-ungesättigten Keton-, Aldehyd- oder Carbonsäure-Duftstoffs neigt und durch die Formel 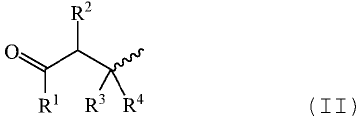
dargestellt ist, in der die Wellenlinie die Lage der Bindung zwischen dem P und S kennzeichnet, R¹ ein Wasserstoffatom, einen C₁- bis C₆-Alkoxylrest oder einen geradkettigen, cyclischen oder verzweigten C₁- bis C₁₅-Alkyl, -Alkenyl- oder -Alkadienylrest, gegebenenfalls substituiert mit einer bis vier C₁- bis C₄-Alkylgruppen, darstellt und
R², R³ und R⁴ unabhängig voneinander ein Wasserstoffatom, einen aromatischen Ring oder einen geradkettigen, cyclischen oder verzweigten C₁- bis C₁₅-Alkyl, -Alkenyl- oder -Alkadienylrest, möglicherweise substituiert mit C₁-bis C₄-Alkylgruppen, darstellen oder zwei oder drei der Gruppen R¹ bis R⁴ zusammen gebunden sind, so dass ein gesättigter oder ungesättigter Ring mit 5 bis 20 Kohlenstoffatomen, einschließlich des Kohlenstoffatoms, an das die Gruppe R¹, R², R³ oder R⁴ gebunden ist, gebildet wird, wobei dieser Ring möglicherweise mit geradkettigen, verzweigten oder cyclischen C₁- bis Cs-Alkyl- oder - Alkenylgruppen substituiert ist;
R eine geradkettige oder verzweigte Alkylgruppe mit 8 bis 15 Kohlenstoffatomen darstellt, die gegebenenfalls eine Carbonsäure- oder eine C₁₋₈-Carbonsäureester-Funktionsgruppe umfasst, die nicht direkt mit dem Schwefelatom verknüpft ist;
umfassend den Schritt, bei dem eine Verbindung der Formel 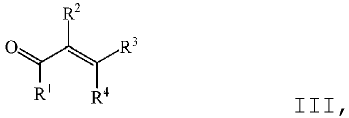
wobei R¹, R², R³ und R⁴ die gleiche Bedeutung wie oben angegeben haben, mit einem Thiol der Formel R-SH, wobei R die gleiche Bedeutung wie oben angegeben hat, umgesetzt wird, **dadurch gekennzeichnet, dass** das Thiol höchstens 50 µg/kg Thiol- oder Thioetherverbindungen mit weniger als 6 Kohlenstoffatomen umfasst.

6. Verfahren nach Anspruch 5, wobei das Thiol der Formel R-SH
a) höchstens 5 µg/kg 1-Propanthiol,
b) höchstens 5 µg/kg 2-Propanthiol,
c) höchstens 5 µg/kg 2-Methyl-1-propanthiol,
d) höchstens 20 µg/kg 2-Methyl-2-propanthiol,
e) höchstens 5 µg/kg 1-Butanthiol und
f) höchstens 10 µg/kg Tetrahydrothiophen umfasst.

7. Verfahren nach einem der Ansprüche 5 bis 6, wobei das Thiol der Formel R-SH keine Spuren von 1-Propanthiol, 2-Propanthiol, 2-Methyl-1-propanthiol, 2-Methyl-2-propanthiol, 1-Butanthiol oder Tetrahydrothiophen aufweist.

8. Verfahren nach einem der Ansprüche 5 bis 7, wobei das Thiol der Formel R-SH vor dem Umsetzen mit der Verbindung der Formel (III) per Destillation aufgereinigt wird.

9. Verfahren nach einem der Ansprüche 5 bis 8, wobei R für eine geradkettige Alkylgruppe mit 10 bis 14 Kohlenstoffatomen stehen kann.

10. Verfahren nach einem der Ansprüche 5 bis 9, wobei es sich bei dem Thiol der Formel R-SH um 1-Dodecanthiol handelt.

11. Verfahren nach einem der Ansprüche 5 bis 10, wobei P für einen aus der Gruppe bestehend aus Formeln (P-1) bis (P-14) ausgewählten Rest in Form eines ihrer Isomere steht: 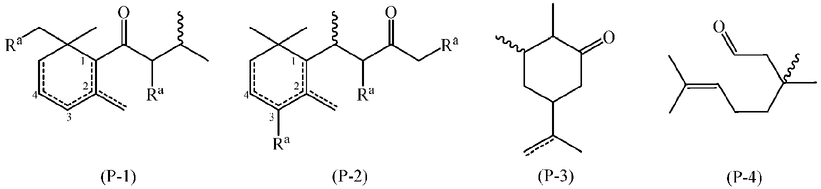 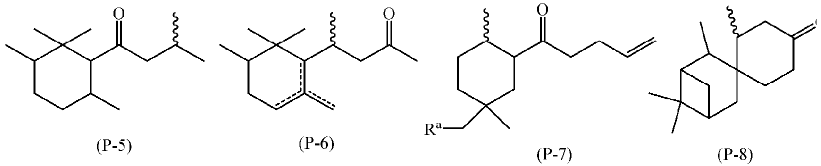 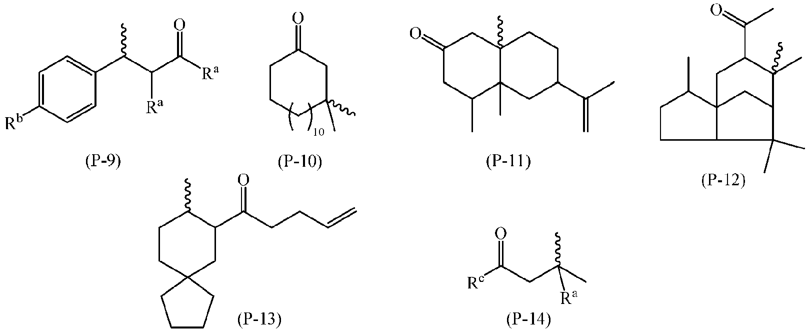 wobei in den Formeln die Wellenlinien die oben bezeichnete Bedeutung haben und die gestrichelten Linien eine Einfach- oder Doppelbindung darstellen, R^{a} für ein Wasserstoffatom oder eine Methylgruppe steht und R^{b} ein Wasserstoffatom, eine Hydroxyl- oder Methoxygruppe oder eine geradkettige oder verzweigte C₁-C₄-Alkylgruppe darstellt und R^{c} ein Wasserstoffatom oder eine eine geradkettige oder verzweigte C₁-C₄-Alkylgruppe darstellt.

12. Verfahren nach einem der Ansprüche 5 bis 11, wobei P für (P-1), (P-2), (P-3), (P-7), (P-13) oder (P-14) steht.

13. Verfahren nach einem der Ansprüche 5 bis 12, wobei die Verbindung der Formel (I) aus der Gruppe 3-(Dodecylthio)-1-(2,6,6-trimethylcyclohex-3-en-1-yl)-butan-1-on, 2-(Dodecylthio)-4-octanon, 3-(Dodecylthio)-1-(2,6,6-trimethylcyclohex-2-en-1-yl)butan-1-on, 4-(Dodecylthio)-4-(2,6,6-trimethylcyclohex-2-en-1-yl)-butan-2-on und 4-(Dodecylthio)-4-(2,6,6-trimethyl-cyclohex-1-en-1-yl)butan-2-on und 4-(Dodecylthio)-4-methylpentan-2-on ausgewählt ist.

14. Verfahren nach einem der Ansprüche 5 bis 13, wobei die Umsetzung zwischen einer Verbindung der Formel (III) und dem Thiol der Formel R-SH in Gegenwart einer Base erfolgt.

15. Stoffzusammensetzung, umfassend eine Verbindung der Formel (I), höchstens 50 µg/kg Thiol- oder Thioetherverbindungen mit weniger als 6 Kohlenstoffatomen und höchstens 1 ppm irgendeiner Verbindung der Formel 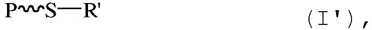 wobei die Wellenlinie die Lage der Bindung zwischen dem P und dem Schwefelatom kennzeichnet, P die gleiche Bedeutung wie in Anspruch 5 bis 14 angegeben hat und R' eine Alkylgruppe mit 1 bis 5 Kohlenstoffatomen darstellt.

## Revendications

1. Procédé de mesure de la quantité de composés thiol ou thioéther ayant moins de 6 atomes de carbone dans le thiol de formule R-SH, R représentant un groupe alkyle linéaire ou ramifié ayant de 8 à 15 atomes de carbone, éventuellement comprenant un groupe fonctionnel acide carboxylique ou ester carboxylique en C₁₋₈, qui n'est pas directement lié à l'atome de soufre, par chromatographie en phase gazeuse-spectrométrie de masse (GC-MS) combinée avec une microextraction sur phase solide (SPME), la SPME étant réalisée avec l'échantillon refroidi jusqu'à une température comprise entre -100 °C et -50 °C.

2. Procédé selon la revendication 1, les composés thiol ou thioéther ayant moins de 6 atomes de carbone étant choisis dans le groupe constitué par 1-propanethiol, 2-propanethiol, 2-méthyl-1-propanethiol, 2-méthyl-2-propanethiol, 1-butanethiol et tétrahydrothiophène.

3. Procédé selon l'une quelconque des revendications 1 à 2, la MS de la GC/MS étant un triple quadrupôle, un quadrupôle à temps de vol (Q-TOF) ou un orbitrap quadripolaire.

4. Procédé selon l'une quelconque des revendications 1 à 3, le mode d'acquisition de données de GC-MS étant la surveillance d'ion sélectionné (SIM) ou la surveillance de réaction sélectionnée (SRM).

5. Procédé pour la préparation d'un composé de formule 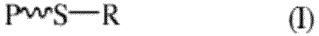
la ligne ondulée indiquant l'emplacement de la liaison entre ledit P et l'atome de soufre ;
P représentant un radical susceptible de générer une cétone, un aldéhyde ou un ester carboxylique α,β-insaturé(e) odorant(e) et étant représenté par la formule 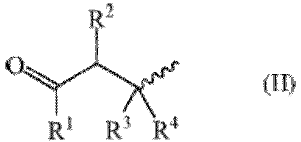
dans laquelle la ligne ondulée indique l'emplacement de la liaison entre ledit P et S ; R¹ représentant un atome d'hydrogène, un radical alcoxyle en C₁₋₆, ou un radical alkyle, alcényle ou alcadiényle linéaire, cyclique ou ramifié en C₁₋₁₅, éventuellement substitué par un à quatre groupes alkyle en C₁₋₄ ; et
R², R³ et R⁴ représentant indépendamment les uns des autres un atome d'hydrogène, un cycle aromatique, ou un radical alkyle, alcényle ou alcadiényle linéaire, cyclique ou ramifié en C₁₋₁₅, possiblement substitué par des groupes alkyle en C₁₋₄ ; ou deux, ou trois, des groupes R¹ à R⁴ étant liés ensemble pour former un cycle saturé ou insaturé ayant 5 à 20 atomes de carbone et comprenant l'atome de carbone auquel lesdits groupes R¹, R², R³ ou R⁴ sont liés, ce cycle étant possiblement substitué par des groupes alkyle ou alcényle linéaires, ramifiés ou cycliques en C₁₋₈ ;
R représentant un groupe alkyle linéaire ou ramifié ayant de 8 à 15 atomes de carbone, éventuellement comprenant un groupe fonctionnel acide carboxylique ou ester carboxylique en C₁₋₈ qui n'est pas directement lié à l'atome de soufre;
comprenant l'étape de mise en réaction d'un composé de formule 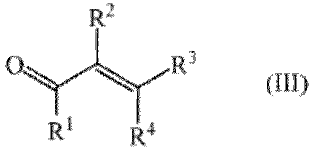
R¹, R², R³ et R⁴ ayant la même signification que définie ci-dessus
avec un thiol de formule R-SH, R ayant la même signification que définie ci-dessus **caractérisé en ce que** le thiol comprend au plus 50 µg/kg de composés thiol ou thioéther ayant moins de 6 atomes de carbone.

6. Procédé selon la revendication 5, le thiol de formule R-SH comprenant
a) au plus 5 µg/kg de 1-propanethiol
b) au plus 5 µg/kg de 2-propanethiol
c) au plus 5 µg/kg de 2-méthyl-1-propanethiol
d) au plus 20 µg/kg de 2-méthyl-2-propanethiol
e) au plus 5 µg/kg de 1-butanethiol ; et
f) au plus 10 µg/kg de tétrahydrothiophène.

7. Procédé selon l'une quelconque des revendications 5 à 6, le thiol de formule R-SH étant exempt de traces de 1-propanethiol, 2-propanethiol, 2-méthyl-1-propanethiol, 2-méthyl-2-propanethiol, 1-butanethiol ou de tétrahydrothiophène.

8. Procédé selon l'une quelconque des revendications 5 à 7, le thiol de formule R-SH étant purifié par distillation avant mise en réaction avec le composé de formule (III).

9. Procédé selon l'une quelconque des revendications 5 à 8, R pouvant être un groupe alkyle linéaire ayant de 10 à 14 atomes de carbone.

10. Procédé selon l'une quelconque des revendications 5 à 9, le thiol de formule R-SH étant le 1-dodécanethiol.

11. Procédé selon l'une quelconque des revendications 5 à 10, P étant un radical choisi dans le groupe constitué par les formules (P-1) à (P-14), sous la forme de l'un quelconque de leurs isomères : 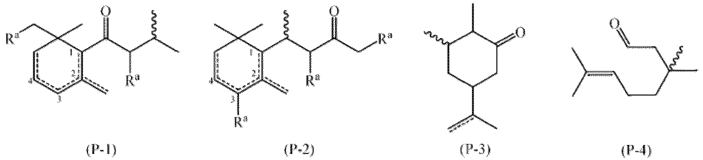 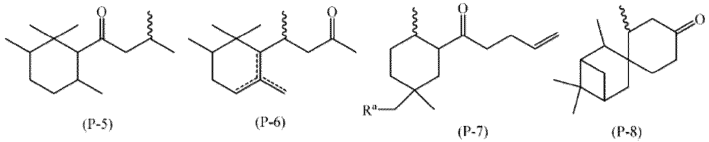 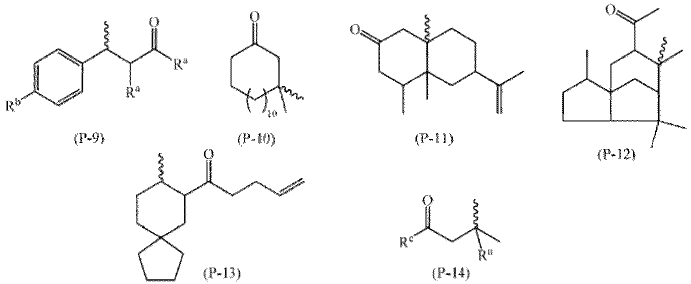 dans lesquelles formules les lignes ondulées possèdent la signification indiquée ci-dessus et les lignes en pointillés représentent une simple ou double liaison, R^{a} étant un atome d'hydrogène ou un groupe méthyle et R^{b} représentant un atome d'hydrogène, un groupe hydroxyle ou méthoxy ou un groupe alkyle linéaire ou ramifié en C₁₋₄ et R^{c} représentant un atome d'hydrogène ou un groupe alkyle linéaire ou ramifié en C₁₋₄.

12. Procédé selon l'une quelconque des revendications 5 à 11, P étant (P-1), (P-2), (P-3), (P-7), (P-13) ou (P-14) .

13. Procédé selon l'une quelconque des revendications 5 à 12, un composé de formule (I) étant choisi dans le groupe de 3-(dodécylthio)-1-(2,6,6-triméthylcyclohex-3-én-1-yl)butan-1-one, 2-(dodécylthio)-4-octanone, 3-(dodécylthio)-1-(2,6,6-triméthylcyclohex-2-én-1-yl)butan-1-one, 4-(dodécylthio)-4-(2,6,6-triméthylcyclohex-2-én-1-yl)butan-2-one et 4-(dodécylthio)-4-(2,6,6-triméthylcyclohex-1-én-1-yl)butan-2-one et 4-(dodécylthio)-4-méthylpentan-2-one.

14. Procédé selon l'une quelconque des revendications 5 à 13, la réaction entre un composé de formule (III) et le thiol de formule R-SH étant conduite en la présence d'une base.

15. Composition de matière comprenant un composé de formule (I), au plus 50 µg/kg de composés thiol ou thioéther ayant moins de 6 atomes de carbone, et au plus 1 ppm d'un quelconque composé de formule 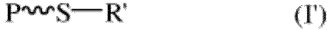 la ligne ondulée indiquant l'emplacement de la liaison entre ledit P et l'atome de soufre ; P ayant la même signification que définie dans les revendications 5 à 14 et R' représentant un groupe alkyle ayant de 1 à 5 atomes de carbone.
